# DEMANDE DE BREVET EUROPEEN

(11) **EP 2 246 016 A1**
(43) Date de publication de la demande: **03.11.2010**
(21) Numéro de dépôt: 09158982.0
(22) Date de dépôt: 28.04.2009
(51) Int. Cl.: A61F 5/56

(54) **Oreiller anti-ronflement**

(71) Demandeur: Oscimed SA, 2300 La Chaux-de-Fonds (CH)
(72) Inventeur: Magnin, Georges, 2314, La Sagne (CH); Magnin, Jacques, 2300, La Chaux-de-Fonds (CH)
(74) Mandataire: GLN

(57) **Abrégé**

La présente invention concerne un oreiller anti-ronflement de forme générale parallélépipédique, de longueur L, de largeur I et d'épaisseur e, l'oreiller comprenant deux faces de dimension L x I, une première (10), inférieure, sur laquelle l'oreiller est destiné à être posé, et une deuxième (12), supérieure, opposée la face inférieure, la face supérieure (12) étant partagée en deux portions (14) sensiblement égales de dimension L/2 x I, chacune desdites portions présente une zone d'appui destinée à recevoir la tête de l'utilisateur, ladite zone définissant
- un bord arrière (14a) situé au milieu de la face supérieure,
- un bord haut (14b) consécutif au bord arrière et destiné à être du côté du sommet du crâne de l'utilisateur,
- un bord avant (14c) opposé au bord arrière, et
- un bord bas (14d), opposé au bord supérieur.

Selon l'invention, la face supérieure (12) comprend une arête centrale en saillie, partageant ladite face en les deux portions. En outre, chaque zone d'appui présente un angle supérieur à 8° en référence à la face inférieure de manière faire basculer la tête de l'utilisateur sur le côté lors des phases de sommeil. En outre, chaque zone d'appui est bordée par un premier rebord (18) au moins dans la portion du bord avant (14c) consécutive au bord haut, de manière à servir d'élément de calage pour le front de l'utilisateur.

## Description

### Description

### OREILLER ANTI-RONFLEMENT

### Domaine technique

La présente invention concerne un oreiller anti-ronflement de forme générale parallélépipédique, de longueur L, de largeur I et d'épaisseur e, l'oreiller comprenant deux faces de dimension L x I, une première, inférieure, sur laquelle l'oreiller est destiné à être posé, et une deuxième, supérieure, opposée la face inférieure.

La face supérieure est partagée en deux portions sensiblement égales de dimension L/2 x I, chacune des portions présente une zone d'appui destinée à recevoir la tête de l'utilisateur, la zone définissant
- un bord arrière situé au milieu de la face supérieure,
- un bord haut consécutif au bord arrière et destiné à être du côté du sommet du crâne de l'utilisateur,
- un bord avant opposé au bord arrière, et
- un bord bas, opposé au bord supérieur.

### Etat de la technique

Lors de la respiration, l'air passe librement par le nez et la bouche, descend dans le pharynx, passe derrière la base de la langue pour rejoindre le larynx, cartilage (pomme d'Adam) contenant les cordes vocales. Le larynx est fixé en haut de la trachée. C'est l'entrée qui mène l'air à l'intérieur des poumons.

Au fond de la bouche, en arrière de la langue, l'espace libre est relativement étroit. L'air doit passer entre la langue, le voile du palais, la luette et la paroi du pharynx. Lors du sommeil, les muscles sont relâchés, hypotoniques. Ils ont alors tendance à s'affaisser ce qui réduit encore le passage. Des conformations anatomiques individuelles, et la forme de la mâchoire inférieure, conditionnent le passage de l'air. Lorsque le voile du palais est long et épais, il réduit l'espace libre. Ces structures molles (paroi pharyngée, voile du palais, base de la langue) s'accolent l'une contre l'autre, et empêchent l'air de passer librement. Il se produit alors une vibration des tissus qui se traduit par le ronflement. Lorsque ce passage est trop réduit, le passage de l'air induit des vibrations, amplifiées par les cavités voisines et par le crâne, qui génèrent le bruit du ronflement.

Le ronflement est non seulement gênant du fait même du bruit généré, mais, dans des formes plus graves du phénomène, il est associé à des apnées obstructives du sommeil, qui peuvent avoir des conséquences plus graves sur la santé de la personne qui en est victime.

Le ronflement peut se traiter de différentes manières, visant à améliorer l'entrée de l'air dans le larynx. On peut utiliser des masques de respiration nocturne insufflant de l'air sous pression. Une telle méthode est efficace, mais lourde et contraignante. On peut également utiliser des orthèses mandibulaires faisant avancer la mandibule. Tout le monde n'apprécie pas de dormir avec un dispositif dans la bouche. En outre, les contraintes que subissent les dents nécessitent que l'utilisation de ces dispositifs soit faite avec vigilance et prudence.

On sait également que dormir sur le côté réduit les ronflements. Divers systèmes ont été proposés de manière à forcer le ronfleur à dormir sur le côté. Par exemple, des pyjamas ont été proposés avec une structure en relief dans le dos, pour empêcher le ronfleur de se mettre sur le dos. Des sacs à dos ont aussi été utilisés dans ce but. Ces systèmes ont évidemment l'inconvénient d'être particulièrement inconfortables et peu pratiques.

La présente invention se rapporte à une autre solution proposée pour permettre à un ronfleur de dormir sur le côté : les oreillers anti-ronflements. Différents modèles existent sur le marché, mais certains n'ont d'anti-ronflements que le nom et d'autres, ne donnent pas toujours satisfaction. La présente invention a pour but de proposer un nouvel oreiller anti-ronflements, garantissant que le ronfleur sera positionné et maintenu sur le côté, tout en lui assurant une position confortable.

### Divulgation de l'invention

De manière plus précise, l'invention porte sur un oreiller anti-ronflement **caractérisé en ce que** la face supérieure comprend une arête centrale en saillie, partageant ladite face, en ce que chaque zone d'appui présente un angle supérieur à 8° en référence à la face arrière, et en ce que la zone d'appui est bordée par un premier rebord au moins dans la portion du bord avant consécutive au bord haut, de manière à servir d'élément de calage pour le front de l'utilisateur.

D'autres caractéristiques d'un oreiller selon l'invention sont mentionnées dans les revendications.

### Brève description des dessins

La présente invention sera mieux comprise à la lecture de la description qui va suivre, faite en référence au dessin annexé, dans lequel:
- la figure 1 est une vue tridimensionnelle d'un oreiller selon l'invention,

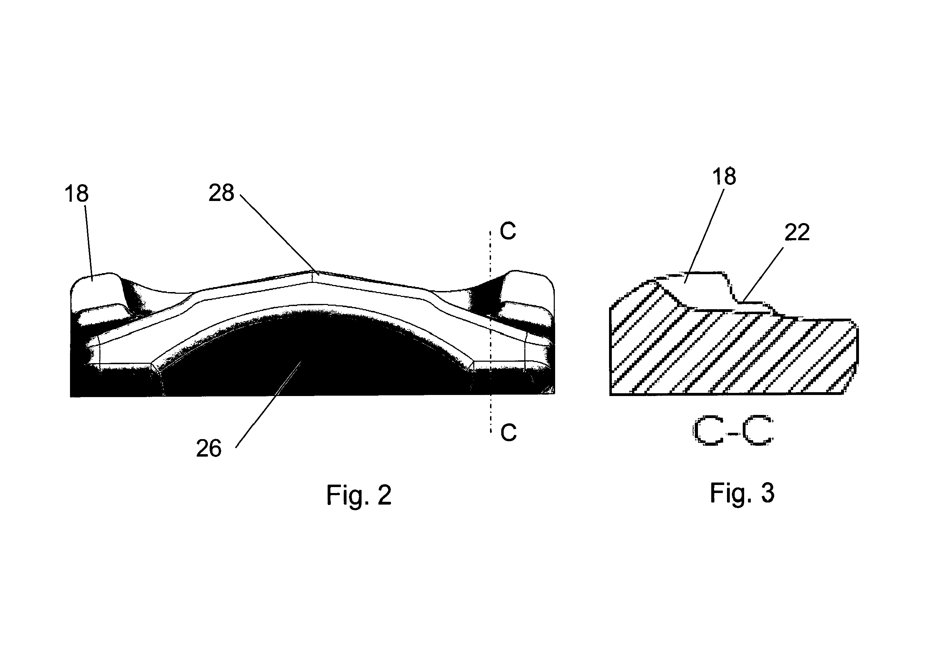

- les figures 2 et 3 sont respectivement des vues de côté et en coupe,
- la figure 4 est une vue de dessus, et
- les figures 5, 6 et 7 sont des vues en coupe supplémentaire d'un oreiller anti-ronflement selon l'invention.

### Mode(s) de réalisation de l'invention

Les figures proposent un mode de réalisation particulier d'un oreiller anti-ronflement selon l'invention. Il est réalisé dans une mousse polymère, typiquement un polyuréthane, supportant le poids de la tête d'un utilisateur sans s'écraser, mais en offrant néanmoins une légère déformation visant à le rendre confortable.

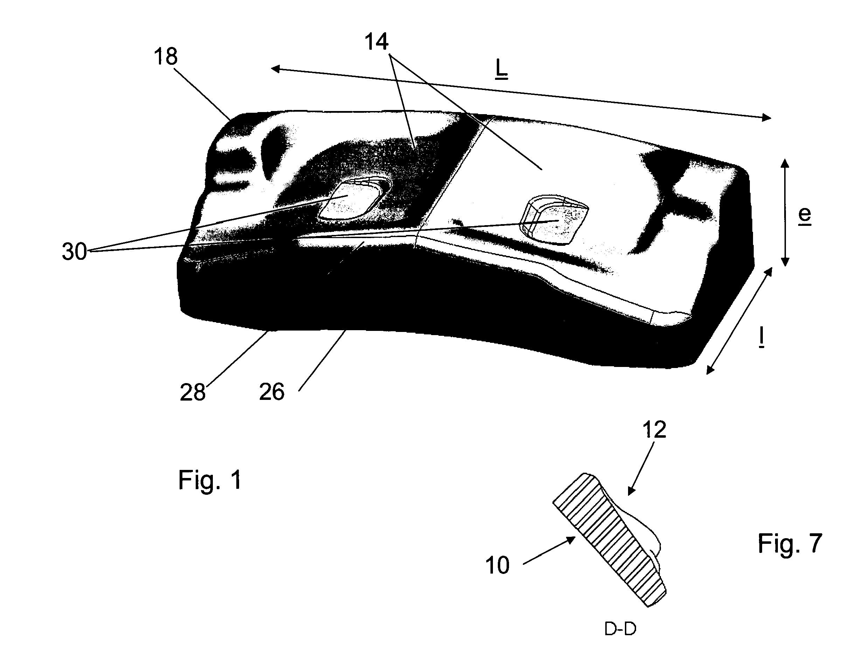

Comme on peut le voir sur la figure 1, l'oreiller représenté est de forme générale parallélépipédique, de longueur L, de largeur I et d'épaisseur e. II comprend deux faces principales, de dimension L x I. Une première 10, inférieure, sur laquelle l'oreiller est destiné à être posé, et une deuxième 12, supérieure, opposée la face inférieure, destinée à recevoir la tête d'un utilisateur.

La face supérieure 12 est partagée en deux portions 14 sensiblement égales de dimension L/2 x I. Chacune des portions ainsi définies est dimensionnée de manière à pouvoir recevoir la tête de l'utilisateur. La surface principale de ces portions définit une zone d'appui destinée plus particulièrement à recevoir la tête de l'utilisateur. De manière à localiser les éléments qui vont être décrits ci-après, on définit, sur chaque zone d'appui :
- un bord arrière 14a situé au milieu de la face supérieure,
- un bord haut 14b consécutif au bord arrière et destiné à être du côté du sommet du crâne de l'utilisateur,
- un bord avant 14c opposé au bord arrière, et
- un bord bas 14d, opposé au bord supérieur.

Selon une première caractéristique importante de l'invention, les deux portions sont définies par une arête centrale 16 en saillie, partageant la face supérieure.

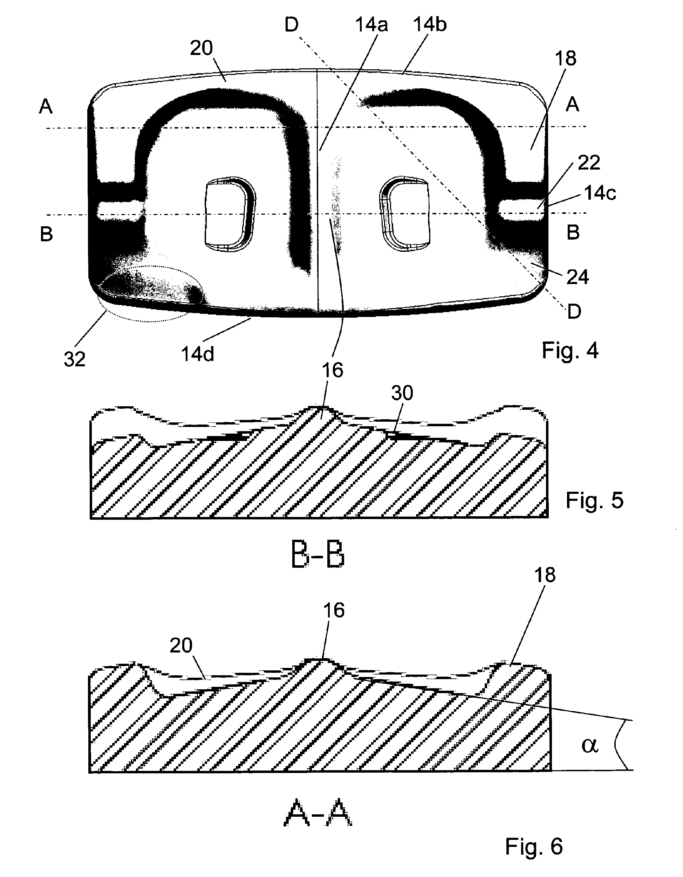

Selon une deuxième caractéristique importante de l'invention, chaque zone d'appui présente un angle α supérieur à 8° en référence à la face arrière. Cet angle, représenté sur la figure 6, peut être compris entre 8 et 25°, de préférence, entre 10 et 12°.

En outre, selon une troisième caractéristique, chaque zone d'appui 14 est bordée par un premier rebord 18 au moins dans la portion du bord avant 14c consécutive au bord haut 14b, de manière à servir d'élément de calage pour le front de l'utilisateur.

Ainsi, grâce à la combinaison de ces trois caractéristiques, un utilisateur va nécessairement positionner sa tête sur l'une ou l'autre des zones d'appui, l'arête centrale 16 l'empêchant de choisir une position intermédiaire. L'inclinaison de cette zone d'appui va automatiquement entraîner la bascule de la tête de l'utilisateur sur le côté, dès lors que le relâchement musculaire provoqué par l'endormissement sera effectif. Ensuite, le premier rebord 18 permet de maintenir la tête dans la position définie par l'angle d'inclinaison de la zone d'appui. De la sorte, l'utilisateur est maintenu dans une position latérale, dans laquelle la tête n'est pas seulement à plat, comme dans un oreiller conventionnel, mais dans laquelle la rotation de la tête est accentuée, ce qui amène à une position inclinée de la tête et ce qui permet de libérer plus efficacement les voies aériennes et donc de diminuer, voire de faire disparaître, les ronflements.

Pour encore améliorer le confort de l'utilisateur et l'efficacité du positionnement de sa tête, on peut également prévoir les dispositions suivantes.

Chaque zone d'appui peut être en outre bordée par un deuxième rebord 20 au niveau de son bord haut 14b, de manière à servir d'élément de butée pour le sommet du crâne de l'utilisateur. La tête de l'utilisateur se trouve ainsi positionnée dans les trois dimensions.

La tête étant ainsi positionnée, le bord avant 14c de chaque zone d'appui peut présenter un premier dégagement 22 destiné à laisser libre le passage du nez et de l'oeil de l'utilisateur, favorisant ainsi sa respiration. Ce premier dégagement 22 est avantageusement ménagé dans le premier rebord 18.

Pour également faciliter la respiration d'un utilisateur, le bord avant 14a de chaque zone d'appui peut présenter, en outre, un deuxième dégagement 24 destiné à laisser libre le passage de la bouche de l'utilisateur. Ce deuxième dégagement 24 peut consister en l'interruption du premier rebord 18.

Pour améliorer le confort de l'utilisateur, la face inférieure peut présenter une creusure 26, destinée à laisser libre le passage de l'épaule de l'utilisateur. Ainsi, l'utilisateur peut loger sa tête au niveau de l'une des portions 14, sans que son épaule vienne buter dans l'oreiller. Au contraire, son épaule peut s'enfiler sous l'oreiller, dans la creusure 26. Egalement dans un but de confort, le bord bas 14d de l'oreiller peut également être muni d'un troisième rebord 28, prolongeant l'arête centrale 16 et suivant sensiblement l'angle formé par la zone d'appui. Ce troisième rebord 28 est particulièrement agencé pour se conformer au cou de l'utilisateur et fournir un appui supplémentaire.

On peut en outre prévoir que chaque zone d'appui présente un renfoncement 30 agencé sensiblement au centre de la zone d'appui, de manière à recevoir l'oreille de l'utilisateur. Ce renfoncement 30 peut présenter un fond parallèle à la face inférieure et débouchant directement au niveau de la zone d'appui.

Enfin, il peut être prévu que le coin de chaque zone d'appui, situé entre le bord avant 14c et le bord inférieur 14d, présente une zone plongeante 32, formant, par rapport à la face inférieure, un angle plus important que le reste de la zone d'appui. Cette zone plongeante 32 permet un relâchement plus important de la mandibule inférieure au cours des phases de sommeil. La mandibule est ainsi très libre et la pesanteur peut s'exercer sur elle et la faire s'avancer, ouvrant ainsi le passage de l'air au niveau du pharynx.

Grâce à ces caractéristiques, l'oreiller anti-ronflement selon l'invention permet, tout en restant confortable, de garantir que l'utilisateur se trouve dans une position latérale, accentuée par rapport à un plan horizontal défini, par exemple, par un matelas, tout en ayant sa tête maintenue. Grâce à la présence des deux zones d'appui, l'utilisateur peut, s'il se retourne, passer d'une position dans laquelle un premier de ses profils est situé dans une première zone, à une position dans laquelle le deuxième de ses profils est situé dans l'autre zone.

## Revendications

1. Oreiller anti-ronflement de forme générale parallélépipédique, de longueur L, de largeur I et d'épaisseur e, l'oreiller comprenant deux faces de dimension L x I, une première (10), inférieure, sur laquelle l'oreiller est destiné à être posé, et une deuxième (12), supérieure, opposée la face inférieure,
la face supérieure (12) étant partagée en deux portions (14) sensiblement égales de dimension L/2 x I, chacune desdites portions présente une zone d'appui destinée à recevoir la tête de l'utilisateur, ladite zone définissant
- un bord arrière (14a) situé au milieu de la face supérieure,
- un bord haut (14b) consécutif au bord arrière et destiné à être du côté du sommet du crâne de l'utilisateur,
- un bord avant (14c) opposé au bord arrière, et
- un bord bas (14d), opposé au bord supérieur,
**caractérisé en ce que** la face supérieure (12) comprend une arête centrale en saillie, partageant ladite face en les deux portions,
**en ce que** chaque zone d'appui présente un angle supérieur à 8° en référence à la face inférieure de manière faire basculer la tête de l'utilisateur sur le côté lors des phases de sommeil,
et **en ce que** chaque zone d'appui est bordée par un premier rebord (18) au moins dans la portion du bord avant (14c) consécutive au bord haut, de manière à servir d'élément de calage pour le front de l'utilisateur.

2. Oreiller selon la revendication 1, **caractérisé en ce que** chaque zone d'appui présente un angle en référence à la face inférieure compris entre 8 et 25°, de préférence, entre 10 et 12°.

3. Oreiller selon l'une des revendications précédentes, **caractérisé en ce que** chaque zone d'appui est en outre bordée par un deuxième rebord (20) au niveau de son bord haut (14b), de manière à servir d'élément de butée pour le sommet du crâne de l'utilisateur.

4. Oreiller selon l'une des revendications précédentes, **caractérisé en ce que** le bord avant (14c) de chaque zone d'appui présente un premier dégagement (22) destiné à laisser libre le passage du nez et de l'oeil de l'utilisateur.

5. Oreiller selon la revendication 4, **caractérisé en ce que** ledit premier dégagement (22) est ménagé dans le premier rebord.

6. Oreiller selon l'une des revendications 4 et 5, **caractérisé en ce que** le bord avant (14c) de chaque zone d'appui présente, en outre, un deuxième dégagement (24) destiné à laisser libre le passage de la bouche de l'utilisateur.

7. Oreiller selon l'une des revendications précédentes, **caractérisé en ce que** la face inférieure (10) présente une creusure (26), destinée à laisser libre le passage de l'épaule de l'utilisateur.

8. Oreiller selon l'une des revendications précédentes, **caractérisé en ce que** le bord bas 14d de chaque zone d'appui est muni d'un troisième rebord (28), prolongeant l'arête centrale 16 et suivant sensiblement l'angle formé par la zone d'appui.

9. Oreiller selon l'une des revendications précédentes, **caractérisé en ce que** chaque zone d'appui présente un renfoncement (30) agencé de manière à recevoir l'oreille de l'utilisateur.

10. Oreiller selon l'une des revendications précédentes, **caractérisé en ce que** le coin de chaque zone d'appui, situé entre le bord avant et le bord inférieur, présente une zone plongeante (32), formant un angle par rapport à la face inférieure, plus important que le reste de la zone d'appui.
